(19) Europäisches Patentamt
European Patent Office
Office européen des brevets

(11) Publication number: **0 160 546**
**A2**

# (12) EUROPEAN PATENT APPLICATION

(21) Application number: 85302985.8

(22) Date of filing: 26.04.85

(51) Int. Cl.[4]: C 07 D 501/46
A 61 K 31/545

(30) Priority: 26.04.84 JP 85900/84
28.05.84 JP 108134/84
19.07.84 JP 151098/84
03.09.84 JP 184986/84
17.10.84 JP 219177/84

(43) Date of publication of application:
06.11.85 Bulletin 85/45

(84) Designated Contracting States:
AT BE CH DE FR GB IT LI LU NL SE

(71) Applicant: YAMANOUCHI PHARMACEUTICAL CO. LTD.
No. 5-1 Nihonbashi-Honcho, 2-chome Chuo-ku
Tokyo(JP)

(72) Inventor: Yamazaki, Atsuki
7-8-506, Shimura 2-chome
Itabashi-ku Tokyo(JP)

(72) Inventor: Shibanuma, Tadao
596-11, Ohyaguchi
Urawa-shi Saitama(JP)

(72) Inventor: Koda, Akio
No. 5-3, Hibarigaoka Kita 4-chome
Hoya-shi Tokyo(JP)

(72) Inventor: Nakano, Kohji
No. 1086-1, Ohaza Sanagaya Shiraoka-cho
Minamisaitama-gun Saitama(JP)

(72) Inventor: Maeda, Tetsuya
No. 14-6, Yotsuya 3-chome
Urawa-shi Saitama(JP)

(72) Inventor: Nagano, Noriaki
No. 13-1, Ayase
Hasuda-shi Saitama(JP)

(72) Inventor: Murakami, Yukiyasu
No. 306-3, Ohmaki
Urawa-shi Saitama(JP)

(72) Inventor: Hara, Ryuichiro
No. 19-11, Honcho 3-chome
Nakano-ku Tokyo(JP)

(74) Representative: Geering, Keith Edwin et al,
REDDIE & GROSE 16 Theobalds Road
London WC1X 8PL(GB)

## (54) Cephalosporin compounds, and their production, and medicaments containing them.

(57) This invention relates to antibacterial cephalosporin compounds, to the production of these compounds, and to medical compositions containing them. The compounds of this invention are those of the general formula I and salts thereof:

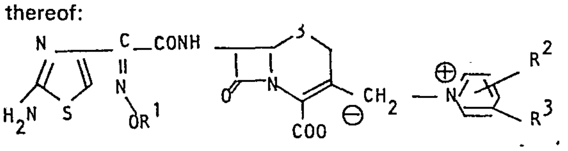

(I)

wherein

$R^1$ represents a lower alkyl group which may be substituted by a carboxyl group;

$R^2$ and $R^3$, which may be the same or different, each represents an amino group, a lower alkyl group, a lower alkylthio group, or a lower alkoxy group which may be substituted by hydroxyl group(s) and or amino group(s), or $R^2$ and $R^3$ combine to form

$$\begin{array}{c} R^4 \\ | \\ -N-A-(CH_2)_m-O- \end{array}$$

wherein

$R^4$ represents a hydrogen atom, a hydroxyl group, or a lower alkoxy group; A represents a direct linkage or a carbonyl group; and m represents an integer of 1 to 2.

EP 0 160 546 A2

0160546

# CEPHALOSPORIN COMPOUNDS, AND THEIR PRODUCTION, AND MEDICAMENTS CONTAINING THEM

This invention relates to antibacterial cephalosporin compounds, to the production of these compounds, and to medical compositions containing them.

The compounds of this invention are those of the general formula I and pharmacologically acceptable salts thereof :

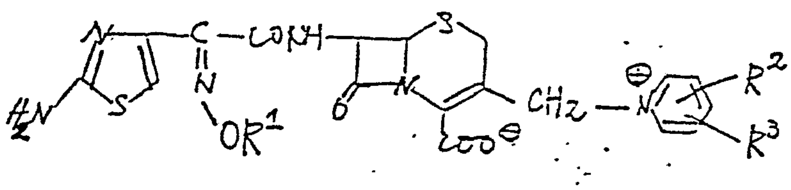

(I)

wherein $R^1$ represents a $C_1$ to $C_5$ alkyl group which may be substituted by a carboxyl group; and $R^2$ and $R^3$ are (a) the same or different and selected from an amino group, $C_1$ to $C_5$ alkyl groups, $C_1$ to $C_5$ alkylthio groups, and $C_1$ to $C_5$ alkoxy groups which may be substituted by hydroxyl group(s) and/or amino group(s), or (b) together form $-N(R^4)-A-(CH_2)_m-O-$ wherein $R^4$ represents a hydrogen atom, a hydroxyl group, or a $C_1$ to $C_5$ alkoxy group, A represents a direct linkage or a carbonyl group, and m is 1 or 2.

Symbols herein have the same significances throughout unless otherwise indicated.

The compounds of this invention shown by formula I show antibacterial activities to various pathogens including several important gram positive or negative bacteria. Some of the compounds of this invention have superior solubility in water, and so are suitable for injection preparations.

Many cephalosporin derivatives having antibacterial activities have been synthesized heretofore, and some are used for medical treatment.

Some cephalosporin compounds having a pyridiniomethyl group as a substituent at the 3-position of the cephalosporin nucleus are known; e.g.

EP. 74,645 discloses some cephalosporin compounds having such a pyridiniomethyl group substituted by an amino or protected amino group, and GB 2,098,216 discloses cephalosporin compounds having such a pyridiniomethyl group substituted by a group which is selected from a $C_1$ to $C_6$ alkyl group, a halogen atom, a hydroxyl group, an alkoxy group, etc.

The term "lower" in the foregoing definition of general formula I and elsewhere herein means a straight or branched carbon chain having 1 to 5 carbon atoms.

Examples of the "lower alkyl group" are methyl, ethyl, propyl, isopropyl, butyl, and pentyl groups. When the lower alkyl group is substituted by a carboxyl group, this carboxyl is in addition to the $C_1$ to $C_5$ chain and may be at any position of the chain; examples of such substituted lower alkyl groups are carboxymethyl, 1-carboxyethyl, 1-carboxy-1-methylethyl, 1-carboxy-1-methylpropyl and 1-carboxy-1-methylbutyl groups. Examples of the "lower alkylthio" in the definition of $R^2$ and $R^3$ are methylthio, ethylthio, propylthio, isopropylthio, butylthio, pentylthio groups. Examples of the "lower alkoxy group which may be substituted by hydroxyl group(s) and/or amino group(s)" in the definitions of $R^2$ and $R^3$ are methoxy, ethoxy, propoxy, isopropoxy, and butoxy groups and such groups substituted by one or two hydroxyl and/or amino groups at any position(s) of the alkoxy group. Examples of the "lower alkoxy group" in the defintion of $R^4$ are the unsubstituted lower alkoxy groups mentioned above for $R^2$ and $R^3$. $R^2$ and $R^3$ may be at any position of the pyridine ring of the pyridinio group. When $R^2$ and $R^3$ combine to form $-\overset{R^4}{N}-A-(CH_2)_m-O-$, $R^2$ and $R^3$ can form with the adjacent pyridine ring of the pyridinio group e.g. a 4-loweralkoxy-3-oxo-3,4-dihydro-2H-pyrido[4,3-b]-1,4-oxazine ring, a 3-oxo-3,4-dihydro-2H-pyrido[4,3-b]-1,4-oxazine ring, a 4-hydroxy-3-oxo-3,4-dihydro-2H-pyrido[4,3-b]-1,4-oxazine, or a 3,4-dihydro-2H-pyrido[4,3-b]-1,4-oxazine.

The compounds of this invention of formula I include geometrical isomers and tautomers; this invention includes all these syn-form and anti-form geometrical isomers and mutual tautomers, alone and in any combination.

The salts of the compounds of this invention of formula I are the pharmacologically acceptable nontoxic salts of the compounds such as nontoxic acid addition salts or base addition salts. Examples of the acid addition salts are pyridinium salts of the formula:

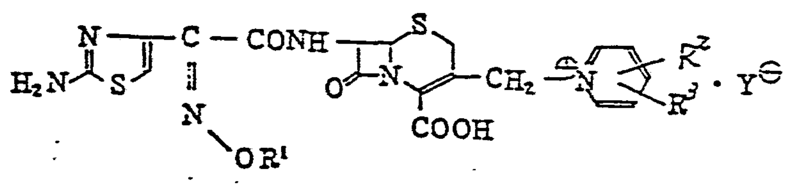

wherein $Y^{\ominus}$ represents an inorganic anion such as a halogen anion ($^-Cl^-$, $I^-$, etc.) or a sulfonic acid anion ($HSO_4^-$, $SO_4^{--}$, etc.); or an organic anion such as an acetate anion ($CH_3COO^-$), a fumarate anion ($HOOC-CH{:}CH\ COO^-$), a citrate anion ($HOOC-CH_2-C(OH)(COOH)-CH_2COO^-$) or a benzenesulfonate anion ($C_6H_5SO_3^-$).

Examples of the base addition salts are salts of the following formula:

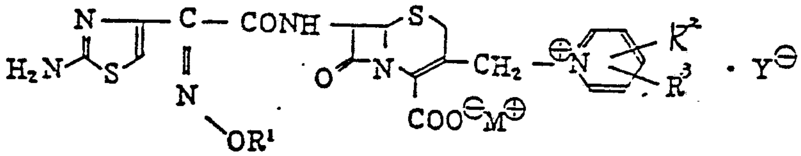

wherein $M^{\oplus}$ represents an alkali metal cation such as $Na^+$ or $K^+$; $NH_4^+$; or an organic base cation such as 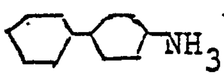$NH_3^+$, 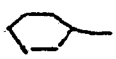$NH_3^+$, $(CH_3)_3NH_3^+$, $(C_2H_5)_3NH_3^+$, $HO\text{-}CH_2CH_2\text{-}NH_3^+$, ornithinium, or lysinium.

The compounds of this invention can be produced by various processes; typical production processes are explained hereinafter:

Process A:

A compound of general formula I can be produced by reacting 7-amino-3-halogenomethyl-$\Delta^3$-cephem-4-carboxylic acid II

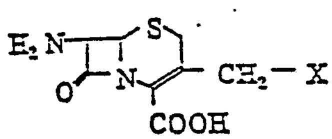

(II)

(wherein X represents a halogen atom) with N,O-bis(tri-loweralkylsilyl)trifluoroacetamide III

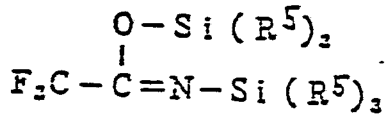

(III)

wherein $R^5$ represents a lower alkyl group.

and substituted pyridine compound IV

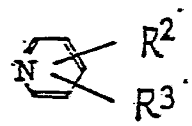

(IV)

and then reacting the formed compound with substituted oxyiminothiazolyl acetic acid compound V or reactive derivative thereof,

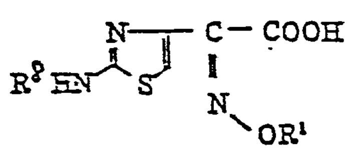

V.

(wherein $R^8$ represents a hydrogen atom or a protective group for an amino group) and then, if necessary, releasing any protective group.

In process A 7-amino-3-halogenomethyl-$\Delta^3$-cephem-4-carboxylic acid

II or a salt thereof can be reacted with N,O-bis(tri-loweralkylsilyl)trifluoroacetamide III and substituted pyridine compound IV in an organic solvent which does not take part in the reaction. When $R_2$ or $R_3$ of compound IV is amino it may be protected by a protection group which (like a protective group $R^8$ of compound V)

may be one usually used in the field of peptide chemistry, practical examples being an acyl group such as a formyl, acetyl, propionyl, tert-butoxycarbonyl, methoxyacetyl, methoxypropionyl, benzyloxycarbonyl, or p-nitrobenzyloxycarbonyl group; an aralkyl group such as a benzyl, benzhydryl, (diphenylmethyl), or trityl group; and a triloweralkylsilyl group such as trimethylsilyl.

Examples of the halogen atoms in the compounds II are a chlorine atom, a bromine atom, an iodine atom.

The compounds III and IV may be reacted with compound II simultaneously or stepwise.

The reaction proceeds easily at room temperature. If performing the reaction stepwise, the first step may be performed at room temperature and the next step under cooling. Various variations of the reaction conditions can be

adopted. Examples of organic solvents which do not take part in the reaction are dichloromethane, acetone, acetonitrile and tetrahydrofuran.

Thus compound A having protective groups ( $(R^5)_3Si-$ ) may be produced, and the resulting reaction

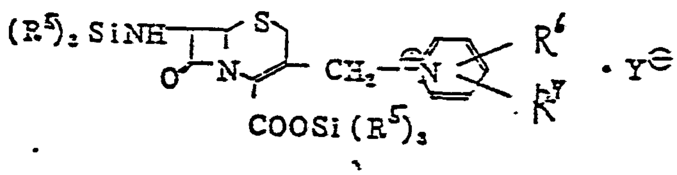

(A)

solution reacted with compound V or reactive derivative thereof. After removing protective groups the desired compound I can be obtained.

The reaction of compounds A and V is usually performed in a solvent under cooling or at room temperature. Any solvent which does not take part in the reaction may be used, and the same solvents as used in the first step are suitable. Examples of the solvents are organic solvents such as dioxane, ether, ethylmethylketone, chloroform, dichloromethane, ethyl acetate, ethyl formate, dimethylformamide, and dimethylsulfoxide; these solvents may be used

alone or in appropriate combinations.

The compound V may be a free carboxylic acid or a reactive derivative thereof. Suitable examples of such reactive derivative are mixed acid anhydrides, acid anhydrides, acid halides, active esters, active amides, and acid azides. When using a free carboxylic acid V it is preferred to use a condensing agent such as N,N'-dicyclo-hexylcarbodiimide or N,N'-diethylcarbodiimide.

When a reactive derivative of a carboxylic acid used, it may be preferred for smooth reaction to operate in the presence of a base. Examples of such a base are inorganic bases such as sodium hydrogencarbonate potassium hydrogencarbonate, sodium carbonate, and potassium carbonate; and organic bases such as trimethylamine, triethylamine and dimethylaniline pyridine.

The removal of an amino protecting group from the reaction product is easily performed, when the group is an aralkyl group (e.g. trityl) or an acyl group, by hydrolysis with acid. As the acid used in this case, formic acid, trifluoroacetic acid and hydrochloric acid, are amongst those preferred. When the group is an triloweralkylsilyl group, its removal is easily performed by contact with water.

Process B:

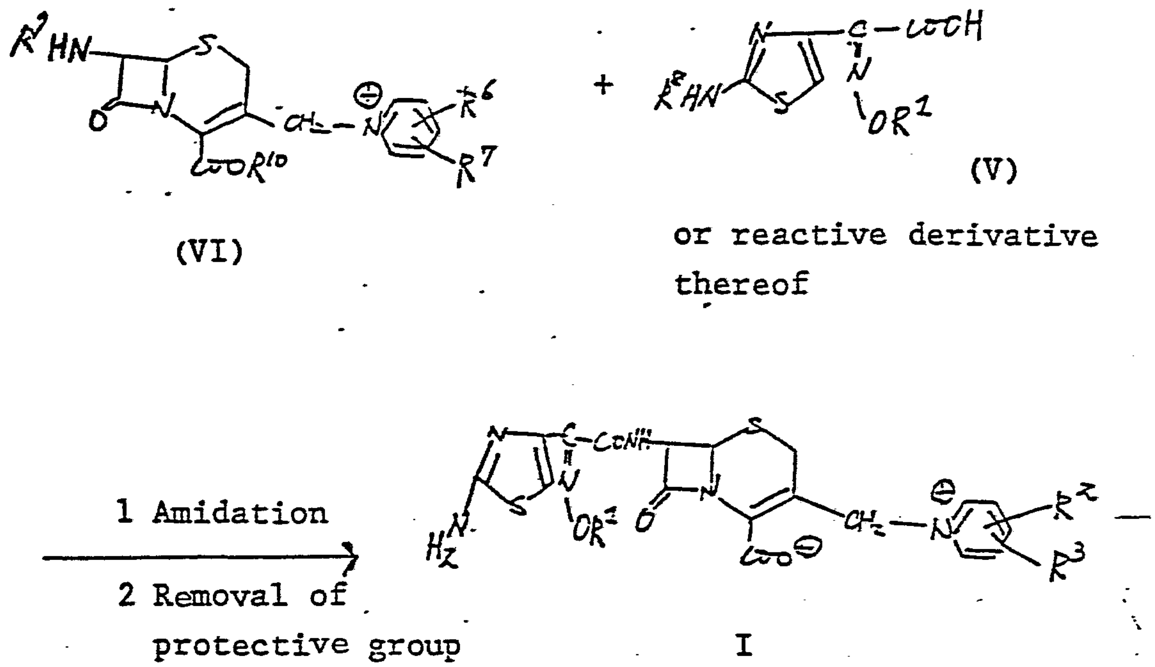


(VI) + (V) or reactive derivative thereof

I

wherein $R^9$ represents a hydrogen atom or a triloweralkylsilyl group, and $R^{10}$ represents a hydrogen atom or a protective group for a carboxyl group.

A compound I can thus be produced by reacting 7-amino-3-cephem derivative VI with substituted oxyiminothiazolylacetic acid V or reactive derivative thereof and then, if necessary, releasing any protective group(s).

Practical examples of protective group for a carboxy group are those which can be released easily under mild conditions, such as trimethylsilyl, benzhydryl, β-methylsulfonylethyl, phenacyl, p-methoxybenzyl, tert-butyl, and p-nitrobenzyl groups.

The reaction can be performed under conditions similar to those for Process A, that is, by adopting the same reaction conditions as for reacting compounds A and V, and then removing protective groups.

Process C:

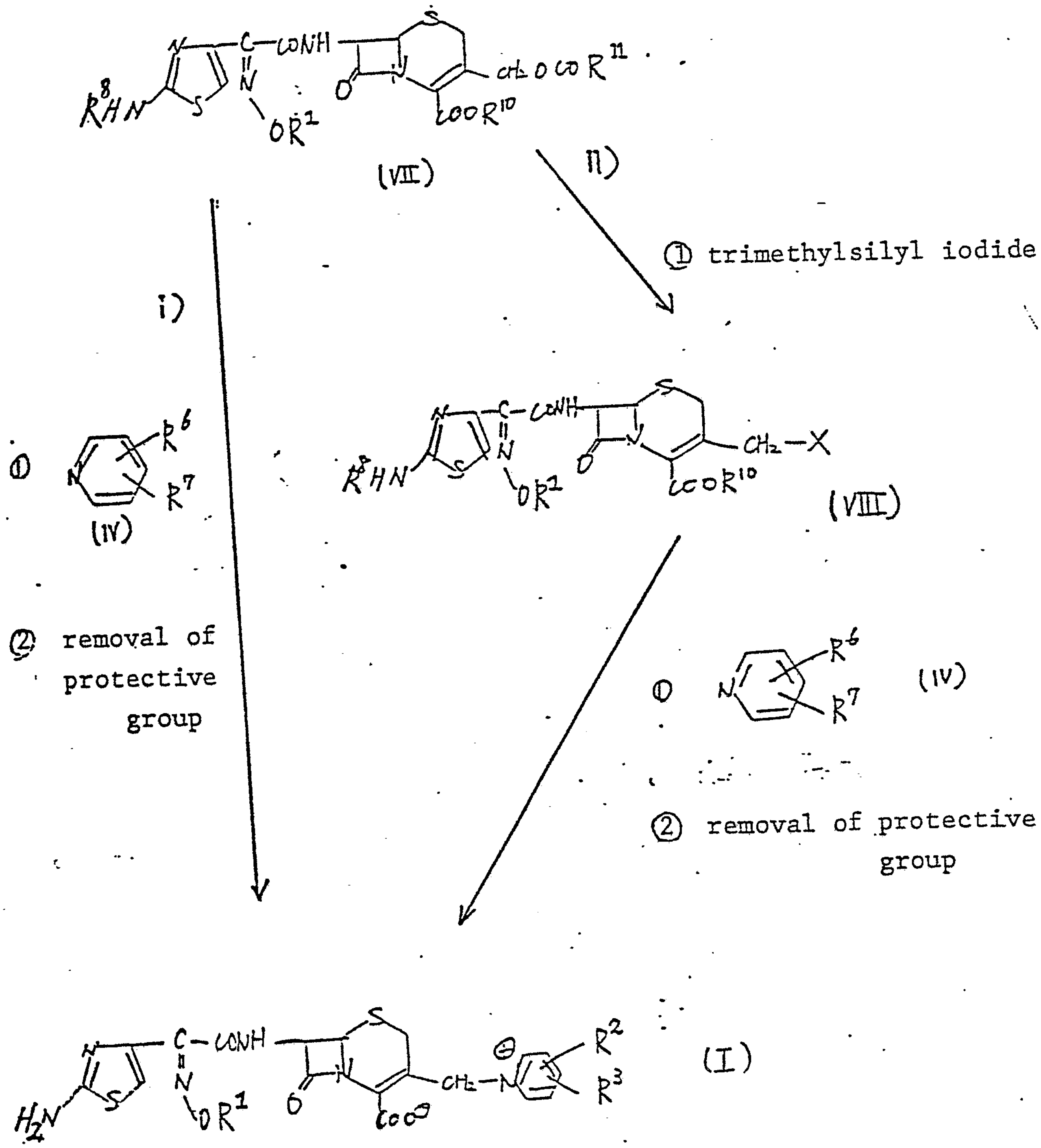


wherein $R^{11}$ represents a lower alkyl group.

Compounds of formula I can thus be prepared (i) by reacting compound VII which may have protective group(s) for the amino group and/or the carboxy group, directly with substituted pyridine compound IV; or (ii) by converting compound VII to the 3-halogenomethylcephalosporin derivative VIII which is then reacted with substituted pyridine compound IV .

This reaction is one for converting the (lower) acyloxy moiety at the 3-position of the cephalosporin nucleus (e.g. acetyloxy, propionyloxy, or butylyloxy) into a substituted pyridinio group

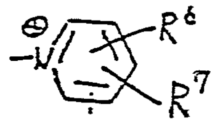

(i) In the case of directly converting the (lower) acyloxy moiety into the pyridinio group, the reaction is usually performed by stirring compound (VII) or a salt thereof and compound (IV) in water or other inert solvent or a mixture thereof.

Examples of inert solvent for this purpose are alcohols such as methanol, ethanol; amides such as dimethylformamide, acetamide; and acetones and acetonitriles. For promoting the reaction, a catalyst such as potassium iodide, potassium bromide, sodium bromide, or potassium thiocyanate may be added to the reaction system in an excess amount. The reaction proceeds easily at room temperature or under heating.

(ii) In the case of obtaining the desired compound (I) via its 3-halogenomethylcephalosporin derivative, the corresponding 3-halogenomethyl derivative (VIII) is first obtained e.g. by reacting compound (VII) (preferably protected by silyl type group) with trimethylsilyl iodide (TMSI) in an inert solvent such as methylene chloride, chloroform, acetonitrile or acetone; the solvent in the silyl-3-halogenomethyl compound-containing solution is distilled away, the concentrate is dissolved in acetonitrile, and excess TMSI is decomposed by adding a small excess of tetrahydrofuran. To the solution thus formed is added the pyridine compound (IV) to provide a silyl derivative of the desired compound.

If desired or necessary, protective group removal and/or salt formation can be effected as previously described following reaction i) or ii).

A salt of the formula I compound can be produced by performing the foregoing production process using a salt of the starting compound, or by applying a salt-forming reaction to the free formula I compound. In the latter case, for example, an alkali metal salt can be produced by adding an n-butanol solution of an alkali 2-ethylhexanoate to the reaction product and then adding thereto an organic solvent of different solvent power, such as ether or ethyl acetate; a salt of an organic base or a basic amino acid can be obtained by adding an equivalent amount or a slight excess of organic base or basic amino acid such as dicyclohexylamine, triethylamine, cyclohexylamine, diethanolamine, arginine or lysine to the reaction product; and an ammonium salt can be obtained by adding aqueous ammonia to the reaction product.

The formula I compounds and salts can be separated and purified in conventional manner, such as extraction with organic solvent, crystallization, or column chromatography.

Compounds of this invention have shown antibacterial activity against various pathogens including several important positive and negative pathogens, and thus are useful as medicaments (especially antibacterial agents), aditives for feeds, and preservatives.

Antibacterial activities (minimum effective inhbitory concentrations) of compounds of formula I (taken from the following Examples) are shown in the following Table.

Table (minimum effective inhibitory concentrations γ/ml)

| Strain | Example No. 2 | 3 | 5 | 6 | 9 | 10 | 11 | 12 | 13 | ceftazidime |
|---|---|---|---|---|---|---|---|---|---|---|
| Staph. epidermidis IID 866 | 1.56 | 0.2 | 0.2 | 0.78 | 0.2 | 6.25 | 0.78 | 1.56 | 0.39 | 3.13 |
| E. coli NIHJ | <0.2 | <0.2 | <0,2 | <0.2 | <0.2 | <0.2 | <0.2 | <0.2 | <0.2 | <0.2 |
| Kleb. pneumoniae ATCC 10031 | <0.2 | <0.2 | <0.2 | <0.2 | <0.2 | <0.2 | <0.2 | <0.2 | <0.2 | <0.2 |
| Ps. aeruginosa NCTC 10490 | 0.39 | 1.56 | 1.56 | 1.56 | 1.56 | 0.39 | 1.56 | 1.56 | 0.78 | 0.78 |
| Serr. marcescense NY-10 | <0.2 | <0.2 | <0.2 | <0.2 | <0.2 | 0.2 | <0.2 | <0.2 | <0.2 | <0.2 |

Some compounds of this invention have low acute toxicity, as proved by animal tests.

Compounds of this invention have superior solubility in water, and aqueous solutions of high concentration can be produced. Thus compounds of this invention have practical value in that injection preparations such as intramuscular injections can be easily provided.

Antibacterial medicaments containing compounds according to the invention may be prepared by conventional methods using conventional carriers or excipients. They may for example be administered parenterally by intravenous or intramuscular injection; orally as tablets, pills, capsules, granules; as liquid preparations; or as suppositories. The appropriate dose is determined in each case considering factors such as the symptom, age and sex of the patient. For an adult a daily total of 250 to 3,000 mg is usually administered in one to four doses.

The invention is illustrated by the following Examples(according to the invention) and Reference Examples. If desired or necessary protective group removal and/or salt formation can be effected in conventional manner as previously described.

Reference Example 1 (raw material for the compound of Example 13)

(a) 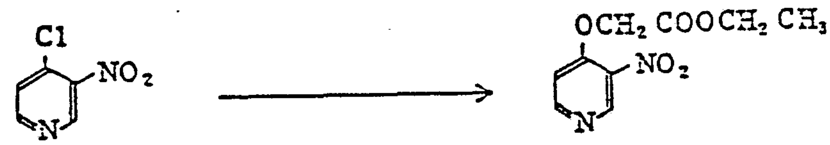

In 200 ml of tetrahydrofuran were dissolved 18.39 g of 4-chloro-3-nitropyridine and 18 g of ethyl glycolate. To the solution formed was added 6.4 g of 60 % oily sodium hydride maintaining the temperature of the solution below 15°C. After stirring the solution at the same temperature, the solvent was distilled off under reduced pressure. To the residue thus obtained were added water and ether, and the ether layer was separated. The ether layer thus separated was extracted with 50 ml of 2N hydrochloric acid three times.To the hydrochloric acid extract was added potassium carbonate to alkalify the extract. The alkalified solution was extracted with ether three times. The ether extract thus obtained wad dried over anhdrous magnesium sulfate, and the solvent was distilled off to give 19.44 g of 4-ethoxycarbonylmethoxy-3-nitropyridine.

EIMS; $(M+H)^+$ 227

NMR ($CDCl_3$; $\partial$ ppm)

1.25, 3H, t, 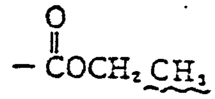

4.25, 2H, q, 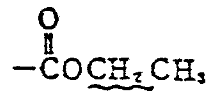

4.82, 2H, s, 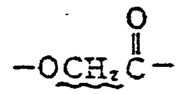

6.86, 1H, d, 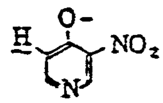

8.57, 1H, d, 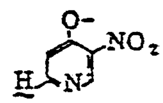

8.97, 1H, s; 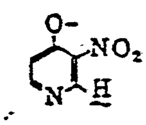

(b) 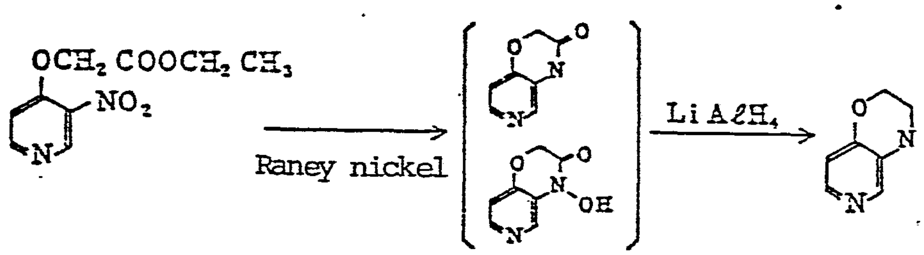


In 30 ml of methanol was dissolved 2.7 g of 4-ethoxycarbonylmethoxy-3-nitropyridine, and about 1.5 ml of Raney nickel was added to the solution. Hydrogen gas was passed through the solution at normal pressure. After the adsorption of hydrogen gas was completed, 100 ml of methanol was added to the reaction mixture, and the catalyst was removed by filtration. The filtrate was concentrated under reduced pressure to give 1.87 g of white powder, a mixture of 4-hydroxy-3-oxo-3,4-dihydro-2H-pyrido[4,3-b]-1,4-oxazine and 3-oxo-3,4-dihydro-2H-pyrido[4,3-b]-1,4-oxazine (85:15). 1.87 g of the mixture was dissolved in 150 ml of dry tetrahydrofuran and after adding thereto 0.93 g of alminium lithium hydride, the formed mixture was refluxed under heating for 2 hours. After cooling the reaction solution, a saturated aqueous sodium sulfate solution and ethyl acetate were added thereto and insolubles were removed by filtration. The filtrate was dried over anhydrous magnesium sulfate, and the solvent was distilled away under reduced pressure to provide 1.28g of pale yellow oily 3,4-dihydro-2H-pyrido[4,3-b]-1,4-oxazine.

NMR (CDCl; δ ppm)

3.42, 2H, t, 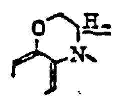

4.29, 2H, t, 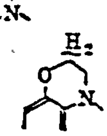

6.64, 1H, d, 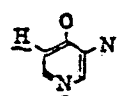

7.80, 1H, d, 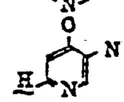

7.88, 1H, s, 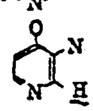

3.84, 1H, broad

Reference Example 2 (raw materials for the compounds of Examples 11 and 12)

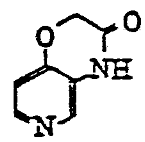 and 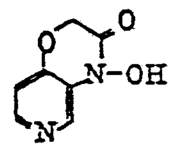

4-ethoxycarbonylmethoxy-3-nitropyridine was treated with hydrogen in a similar manner to Reference Example 1(b), and white powder obtained was recrystallized from water to provide white crystals of 4-hydroxy-3-oxo-3,4-dihydro-2H-pyrido-[4,3-b]-1,4-oxazine.

NMR (DMSO-$d^6$, $\delta$ppm)

4.92, 2H, s, 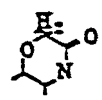, 7.03, 1H, d, 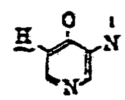

8.19, 1H, d, 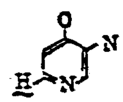, 8.40, 1H, s, 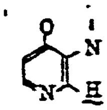

From the mother liquor was obtained 3-oxo-3,4-dihydro-2H-pyrido[4,3-b]-1,4-oxazine.

NMR (DMSO-$d^6$, $\delta$ppm)

4.72, 2H, s, 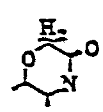, 6.97, 1H, d, 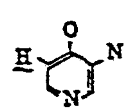

8.07, 1H, d, 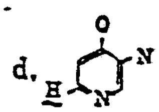, 8.08, 1H, s, 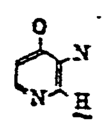

Reference Example 3 (raw material for Example 10 compound)

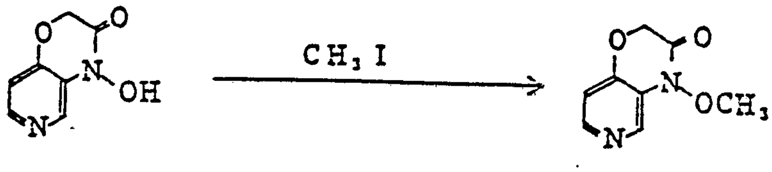


810 mg of 4-hydroxy-3-oxo-3,4-dihydro-2H-pyrido-[4,3-b]-1,4-oxazine was dissolved in a mixture of 4.9 ml of 1N-aqueous solution of sodium hydroxide and 5 ml of acetone. After adding thereto 0.305 ml of methyl iodide, the mixture was stirred for 1 hour at room temperature.

Acetone was distilled away under reduced pressure. To the residue was added a saturated aqueous sodium chloride solution and ethyl acetate. The separated organic layer was taken, dried over anhydrous magnesium sulfate, and the solvent was distilled away under reduced pressure to provide 410 mg of pale yellow powder of 4-methoxy-3-oxo-3,4-dihydro-2H-pyrido[4,3-b]-1,4-oxazine.

mp. 115～119℃

NMR ($CDCl_3$; δppm)

4.02, 3H, s, 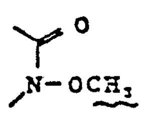

4.80, 2H, s, 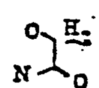

6.92, 1H, d, 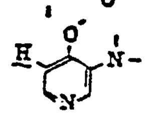

8.28, 1H, d, 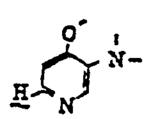

8.46, 1H, s, 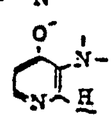

Example 1

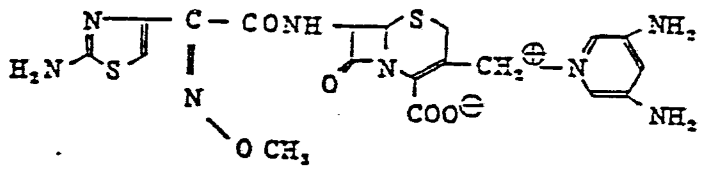

In 30 ml of dichloromethane was suspended 1.02 g of 7-amino-3-iodomethyl-$\Delta^3$-cephem-4-carboxylic acid and after adding thereto 1.7 ml of N,O-bis(trimethylsilyl)-trifluoroacetamide, the mixture was stirred at room temperature for 30 minutes to provide a clear "Solution A".

In 10 ml of dichloromethane was suspended 327 mg of 3,5-diaminopyridine and after adding thereto 4.9 ml of N,O-bis(trimethylsilyl)trifluoroacetamide, the mixture was heated to 30-35°C to provide "Solution B", which was cooled to room temperature.

After adding Solution B to Solution A, the mixture was stirred at room temperature for 4 hours. The reaction solution was cooled to -50°C, and 1.2 ml of pyridine was added thereto to provide a "Solution C".

In 10 ml of dichloromethane was suspended 1.33 g of (Z)-α-(2-tritylamino-4-thiazolyl)-α-(methoxyimino)-acetic acid and after adding thereto 624 mg of phosphorus

pentachloride, the mixture was stirred for 15 minutes to provide a "Solution D". Solution D was added dropwise to Solution C and the temperature of the mixture was increased to -15°C over a period of 15 minutes. The reaction mixture was stirred at the same temperature for 10 minutes. After adding to the solution 8 ml of 1 N hydrochloric acid, 10 ml of water and 10 ml of tetrahydrofuran and stirring the resultant mixture under ice-cooling for 10 minutes, dichloromethane and tetrahydrofuran were distilled away under reduced pressure. To the residue was added 100 ml of water,and powder thus formed was collected by filtration, washed with water, and dried to provide a crude product of the desired compound having protective groups. After adding thereto 30 ml of trifluoroacetic acid under ice-cooling and then adding 3 ml of water, the resultant mixture was stirred at room temperature for 1 hour. Insoluble materials were removed by filtration, and the reaction solution was concentrated under reduced pressure. To the residue was added 100 ml of ether, and the powder thus formed was collected by filtration, and dried to provide 1g of a crude product. The crude product was suspended in 200 ml of water, and 8 ml of 1 N hydrochloric acid was added to dissolve the suspension. The aqueous solution thus formed was adsorbed on Dia HP-20 (made by Mitsubishi Chemical Industries Ltd). Then the product was eluted first with water and then with a mixed solution of water and methanol (mixing ratio of 9:1) and then with a mixed solution of water and methanol

(mixing ratio of 8:2). The fractions containing the desired product were collected, concentrated, and lyophilized to provide 96 mg of (Z)-7-[α-(2-amino-4-thiazolyl)-α-(methoxyimino)acetamido]-3-(3,5-diamino-1-pyridiniomethyl)-$\Delta^3$-cephem-4-carboxylate.

NMR ( DMSO-$d_6$ )

δ : ppm 3.24 ( 2 H, q , 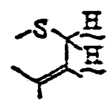 )

3.80 ( 3 H, s , 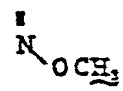 )

5.05 ( 1 H, d , 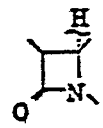 )

5.17 ( 2 H, q , 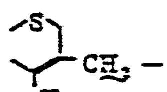 )

5.64 ( 1 H, q , 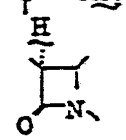 )

6.63 ( 1 H, s , 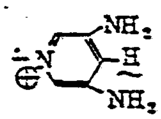 )

6.72 ( 1 H, s , 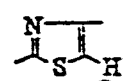 )

7.68 ( 2 H, s , 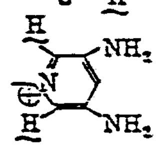 )

Example 2

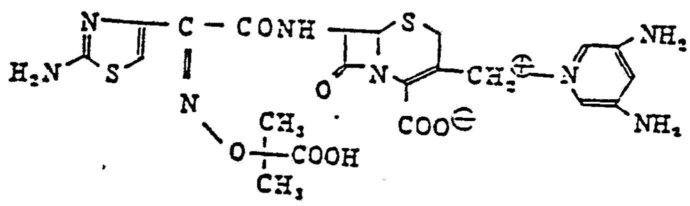

In 30 ml of dichloromethane was suspended 680 mg of 7-amino-3-iodomethyl-$\Delta^3$-cephem-4-carboxylic acid and after adding thereto 1.2 ml of N,O-bis(trimethylsilyl)-trifluoroacetamide, the resultant mixture was stirred at room temperature for 30 minutes to provide a clear "Solution A".

218 mg of 3,5-diaminopyridine was suspended in 10 ml of dichloromethane and after adding thereto 3.4 ml of N,O-bis(trimethylsilyl)trifluoroacetamide, the mixture was heated to 30 - 35°C to dissolve the mixture, and the resultant "Solution B" was cooled to room temperature.

Solution B was added to Solution A and after stirring the mixture at room temperature for 4 hours, the mixture was cooled to -50°C, and 0.8 ml of pyridine was added thereto to provide a "Solution C".

1.14 g of (Z)-α-(2-tritylamino-4-thiazolyl)-α-(1-tert-butoxycarbonyl-1-methylethoxyimino)-acetic acid was suspended in 5 ml of dichloromethane and after adding thereto 416 mg of phosphorus pentachloride under ice-cooling, the resultant mixture was stirred for 15 minutes to provide a "Solution D".

Solution D was added dropwise to Solution C and the temperature of the solution was increased to -15°C over a period of 15 minutes. The solution was stirred at the same temperature 10 minutes and after adding thereto 6 ml of 1 N hydrochloric acid, 8 ml of water and 8 ml of tetrahydrofuran, the mixture was stirred for 10 minutes under ice-cooling, and dichloromethane and tetrahydrofuran were distilled away under reduced pressure. To the residue was added 70 ml of water, and the powder thus formed was collected by filtration, washed with water, and dried to provide a crude product of the desired compound having protective groups. To the crude product obtained was added 20 ml of trifluoroacetic acid under ice-cooling, and the resultant mixture was stirred at room temperature for 1 hour. To the mixture was added 2 ml of water followed by further stirring 1 hour at room temperature. Insoluble materials were removed by filtration, and the reaction solution was concentrated under reduced pressure. To the residue was added 80 ml of ether, and the powder thus formed was collected by filtration, and dried to provide 1 g of a crude product.

The crude product was suspended in 200 ml of water, and 6 ml of 1 N hydrochloric acid was added thereto to dissolve the product. The aqueous solution thus formed

 0160546

was adsorbed on Diaion HP-20, and the product was eluted first with water and then with a mixture of water and methanol (9:1) and then with a mixture of water and methanol (8:2). The fractions containing the desired compound were collected, concentrated, and lyophilized to provide 69 mg of (Z)-7-[α-(2-amino-4-thiazolyl)-α-(1-carboxy-1-methylethoxyimino)acetamido]-3-(3,5-diamino-1-pyridiniomethyl)-$\Delta^3$-cephem-4-carboxylate.

NMR (DMSO-$d_6$)

1.42 (6 H, s, 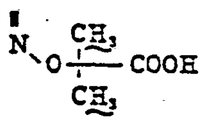 )

3.26 (2 H, q, 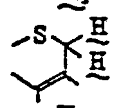 )

5.08 (1 H, d, 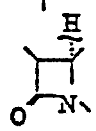 )

5.28 (2 H, q, 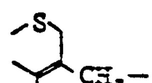 )

5.72 (1 H, q, 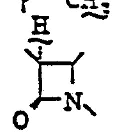 )

6.63 (1 H, s, 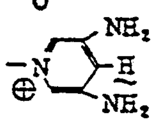 )

6.72 (1 H, s, 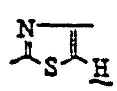 )

7.65 (2 H, s, 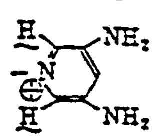 )

Example 3

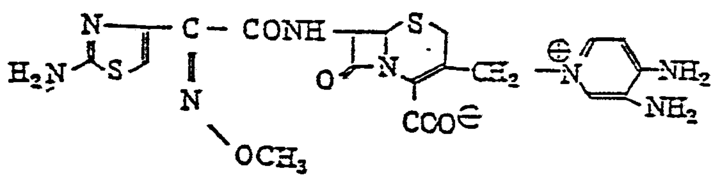

In 40 ml of dichloromethane was suspended 1.36 g of 7-amino-3-iodomethyl-$\Delta^3$-cephem-4-carboxylic acid and after adding thereto 2.4 ml of N,O-bis(trimethylsilyl)trifluoroacetamide, the resultant mixture was stirred at room temperature for 30 minutes to form a complete solution. To the solution was added an aqueous solution which was prepared by suspending 436 mg of 3,4-diaminopyridine in 10 ml of dichloromethane and adding thereto 2.4 ml of N,O-bis(trimethylsilyl)trifluoroacetamide to dissolve the suspension, and the solution was stirred at room temperature for 4 hours. The reaction solution was cooled to -50°C.

and 1.6 ml of pyridine was added thereto to provide a "Solution A".

1.77 g of (Z)-α-(2-tritylamino-4-thiazolyl)-α-(methoxyimino)acetic acid was suspended in 10 ml of dichloromethane and after adding thereto 832 mg of phosphorus pentachloride under cooling, the mixture was stirred for 15 minutes to form a "Solution B".

Solution B was added dropwise to Solution A, the temperature of the solution was increased to -15°C over a period of 10 minutes, and the solution was stirred at the same temperature for 10 minutes. To the solution was added 8 ml of 1 N hydrochloric acid, 10 ml of water and 10 ml of tetrahydrofuran and after stirring the mixture under ice-cooling for 10 minutes, dichloromethane and tetrahydrofuran was distilled away under reduced pressure. To the residue was added 100 ml of water, and the powder formed was collected by filtration, washed with water, and dried to provide a crude product of the desired compound having protective groups. After adding thereto 30 ml of trifluoroacetic acid and then 3 ml of water under ice-cooling, the resultant mixture was stirred at room temperature for 1 hour. Insoluble materials were removed by filtration, and the reaction solution was concentrated under reduced pressure. To the residue was added 100 ml of ether, and the formed powder was collected by filtration and dried to provide 1.3 g of a crude product. The product was suspended in 200 ml of water, and 6 ml of 1 N hydrochloric acid was added thereto to form an aqueous solution. The aqueous solution was adsorbed on Diaion HP-20, and eluted first with water, then with water-methanol (9:1), and further with water-methanol (8:2). The fractions containing the desired product were collected, concentrated, and lyophilized to provide 221 mg of (Z)-7-[α-(2-amino-4-thiazolyl)-α-(methoxyimino)acetamido]-3-(3,4-diamino-1-pyridiniomethyl)-$\Delta^3$-cephem-4-carboxylate.

NMR (DMSO-$d_6$)

δ : ppm 3.16 (2H, q, 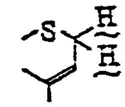 )

3.78 (3H, s, 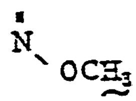 )

4.95 (2H, q, 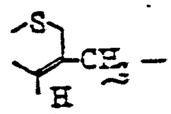 )

5.03 (1H, d, 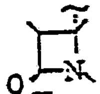 )

5.61 (1H, q, 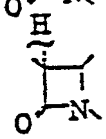 )

6.69 (1H, s, 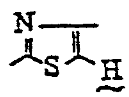 )

6.74 (1H, d, 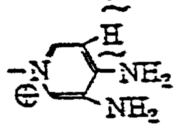 )

7.92 ~ 8.10 (2H, m, 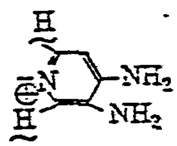 )

Example 4



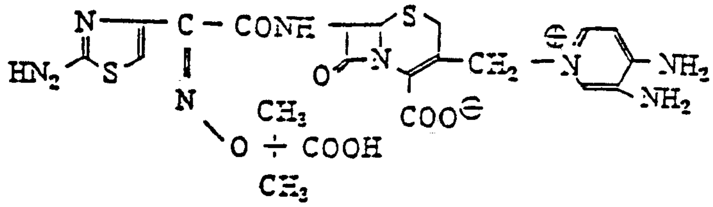

In 30 ml of dichloromethane was suspended 1.02 g of 7-amino-3-iodomethyl-$\Delta^3$-cephem-4-carboxylic acid and after adding thereto 1.7 ml of N,O-bis(trimethylsilyl)-trifluoroacetamide, the mixture was stirred at room temperature for 30 minutes to form a complete solution. To the solution was added another solution which was prepared by suspending 327 mg of 3,4-diaminopyridine in 10 ml of dichloromethane and adding thereto 1.7 ml of N,O-bis(tri-methylsilyl)trifluoroacetamide to dissolve the suspension, and the mixture was stirred at room temperature for 4 hours. The reaction solution was cooled to -50°C, and 1.2 ml of pyridine was added thereto to form a "Solution A".

1.7 g of (Z)-$\alpha$-(2-tritylamino-4-thiazolyl)-$\alpha$-(1-tert-butoxycarbonyl-1-methylethoxyimino)-acetic acid was suspended in 10 ml of dichloromethane and after adding thereto 624 mg of phosphorus pentachloride under ice-cooling, the mixture was stirred for 15 minutes to form a "Solution B".

Solution B was added to Solution A, and the temperature of the solution was increased to -15°C over a period of 10 minutes. The solution was stirred for 10 minutes at the same temperature. After adding to the solution 8 ml of 1N hydrochloric acid, in 10 ml of water and 10 ml of tetrahydrofuran,

the mixture was stirred for 10 minutes under ice-cooling, and dichloromethane and tetrahydrofuran were distilled away under reduced pressure. To the residue was added 100 ml of water, and the formed powder was collected by filtration, washed with water, and dried to provide a crude product of the desired compound having protective groups. After adding thereto 30 ml of trifluoroacetic acid under ice-cooling, the mixture was stirred at room temperature for 1 hour. To the reaction solution was added 3 ml of water, and the mixture was stirred for 1 hour at room temperature.

Insoluble materials were removed by filtration, and the reaction solution was concentrated under reduced pressure. To the residue was added 100 ml of ether, and the formed powder was collected by filtration and was dried to provide a 1.6 g of a crude product.

The crude product was suspended in 200 ml of water, and 8 ml of 1 N hydrochloric acid was added thereto to form a solution. The solution was adsorbed on Diaion HP-20, and eluted first with water, then with water-methanol (9:1), and then with water-methanol (8:2). The fractions containing the desired product were concentrated , and lyophilized to provide252 mg of (Z)-7-[α-(2-amino-4-thiazolyl)-α-(1-carboxy-1-methylethoxyimino)acetamido]-3-(3,4-diamino-1-pyridiniomethyl-$\Delta^3$-cephem-4-carboxylate.

NMR (DMSO-$d_6$)

δ : ppm 1.41 (6H, s, 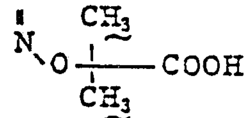 )

3.19 (2H, q, 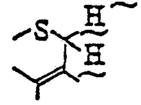 )

4.96 (2H, q, 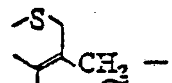 )

5.04 (1H, d, 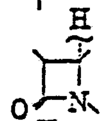 )

5.70 (1H, q, 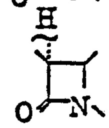 )

6.67 (1H, s, 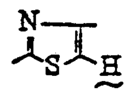 )

6.70 (1H, d, 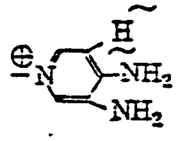 )

7.80 ~ 8.04 (2H, m, 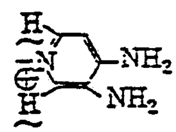 )

Example 5

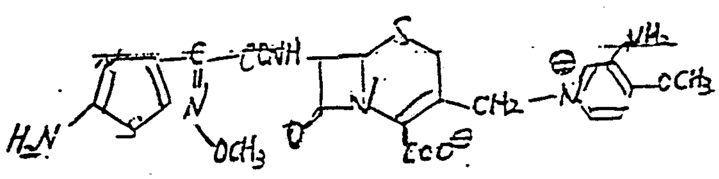

i) In 20 ml of dichloromethane was spended 680 mg of 7-amino-3-iodomethyl-$\Delta^3$-cephem-4-carboxylic acid and after adding thereto 1.15 ml of N,O-bis(trimethylsilyl)trifluoroacetamide, the mixture was stirred at room temperature for 10 minutes to form a solution. To the solution was added 261 mg of 3-amino-4-methoxypyridine, and the mixture was stirred at room temperature for 4.5 hours to provide

a solution containing trimethylsilyl 7-trimethylsilylamino-3-(3-amino-4-methoxypyridiniomethyl)-$\Delta^3$-cephem-4-carboxylate iodide.

ii) In 10 ml of dichloromethane was suspended 886 mg of (Z)-α-(2-tritylamino-4-thiazolyl)-α-methoxyiminoacetic acid and after cooling the suspension to 3 to 4°C and adding thereto 416 mg of phosphorus pentachloride, the mixture was stirred for 15 minutes at 3-4°C to form a "Solution A".

A solution containing trimethylsilyl 7-trimethylsilylamino-3-(3-amino-4-methoxypyridiniomethyl)-$\Delta^3$-cephem-4-carboxylate iodide obtained as at 5i) above was cooled to -40°C and after adding thereto 0.8 ml of pyridine and then adding dropwise Solution A, the temperature of the mixture was increased to -15°C over a period of 15 minutes. To the reaction mixture were added 2 ml of water, 5 ml of tetrahydrofuran and 4 ml of 1 N hydrochloric acid, and the resultant mixture was stirred under ice-cooling for 10 minutes. Dichloromethane and tetrahydrofuran were distilled away under reduced pressure. To the residue was added 100 ml of water, and precipitates thus formed were collected by filtration. The precipitates were washed with water, and dried to provide 1.74 g of a crude product of the desired compound having protective groups. After adding thereto 20 ml of trifluoroacetic acid under ice-cooling and stirring the mixture for 15 minutes at room temperature, insoluble materials were removed by filtration. To the filtrate were added 10 ml of water under ice-cooling, and the mixture was stirred for 1 hour

at room temperature. The reaction mixture was concentrated under reduced pressure, and ether was added to the residue. The formed powder was collected by filtration to provide 1.15 g of a crude product. The crude product was dissolved in 50 ml of water and 5 ml of 1N-hydrochloric acid, the solution was subjected to column chromatography on Diaion HP-20, and the product was eluted first with water and then with mixed solutions of water and methanol (successively changing the mixing ratio from 95 : 5 to 75 : 25). The fractions containing the desired product were collected, concentrated, and lyophilized to provide 279 mg of (Z)-7-[α-(2-amino-4-thiazolyl)-α-methoxyiminoacetamido]-3-(3-amino-4-methoxypyridiniomethyl)-$\Delta^3$-cephem-4-carboxylate.

NMR (DMSO-$d^6$) δppm

| | | | |
|---|---|---|---|
| 2.84～3.60 | 2H, | q, | 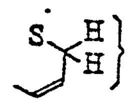 |
| 3.80 | 3H, | s, | 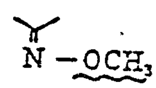 |
| 4.05 | 3H, | s, | 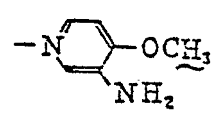 |
| 4.80～5.60 | 2H, | q, | 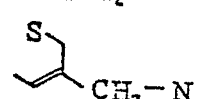 |
| 5.06 | 1H, | d, | 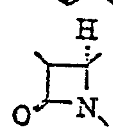 |
| 5.65 | 1H, | dd, | 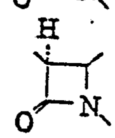 |
| 6.22 | , | s, | broad |
| 6.72 | 1H, | s, | 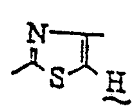 |

| | | | |
|---|---|---|---|
| 7.20 | , | s, | broad |
| 7.44 | 1H, | d, | 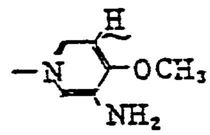 |
| 8.44 | 1H, | s, | 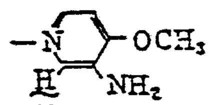 |
| 8.54 | 1H, | d, | 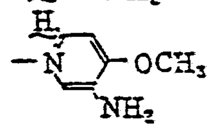 |
| 9.53 | 1H, | d, | $-CONH-$ |

IR (KBr) $cm^{-1}$

2900～3600

1765

1610

1530

FABMS $(M+H)^{+}$ 520

Solubility in water: above 23(W/V)%

0160546

Example 6

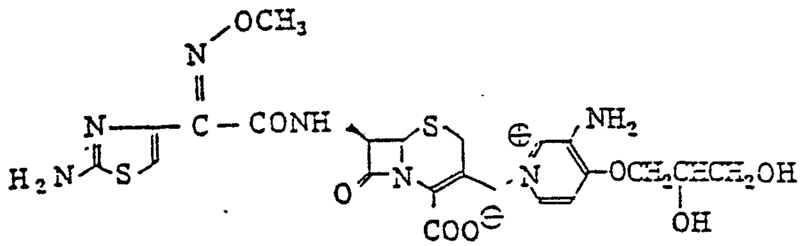

i) In 20 ml of dichloromethane was suspended 680 mg of 7-amino-3-iodomethyl-$\Delta^3$-cephem-4-carboxylic acid and after adding thereto 1.15 ml of N,O-bis(trimethylsilyl)-trifluoroacetamide, the mixture was stirred for 10 minutes at room temperature to provide a solution.To the solution was added 538 mg of 3-amino-4-[(2,2-dimethyl-1,3-dioxolan-4-yl )methoxy]pyridine, and the mixture was stirred for 3.75 hours at room temperatureto provide a solution containing trimethylsilyl 7-trimethylsilyl-amino-3-[3-amino-4-(2,2-dimethyl-1,3-dioxolan-4-yl)-methoxypyridiniomethyl]-4-carboxylate iodide. After cooling the solution to -30°C and adding thereto 0.8 ml of pyridine, and further adding to the mixture dropwise Solution A obtained in Example 5 ii), the temperature of the mixture was increased to -15°C over a period of 15 minutes. To the reaction solution was aded 2 ml of water, 5 ml of tetrahydrofuran and 4 ml of 1 N hydrochloric acid, and the mixture was stirred for 10 minutes under ice-cooling. Dichloromethane and tetrahydrofuran were distilled away under reduced pressure. To the residue was added 100 ml of water, and the precipitates thus formed were collected by filtration, washed with water, and dried to provide 1.78 g of a crude product of the desired

compound having protective groups. To the crude product was added 20 ml of trifluoroacetic acid under ice-cooling and after stirring the mixture for 15 minutes at room temperature, insoluble materials were removed by filtration. To the filtrate was added 10 ml of water under ice-cooling, and the mixture was stirred for 1 hour at room temperature. The mixture was concentrated under reduced pressure and after adding ether to the residue, the formed powder was collected by filtration to provide 1.2 g of a crude product. To the product were added 50 ml of water and 5 ml of 1 N hydrochloric acid to dissolve it, and the solution was subjected to column chromatography on Diaion HP-20. The product was eluted first with water and then with mixtures of water and methanol while successively changing the mixing ratio from 95:5 to 80:20. The fractions containing the desired product were collected, concentrated, and then lyophilized to provide 289 mg of (Z)-7-[α-(2-amino-4-thiazolyl)-α-methoxyiminoacetamido]-3-[3-amino-4-(2,3-dihydroxypropyloxy) pyridiniomethyl]-$\Delta^3$-cephem-4-carboxylate.

NMR (DMSO - $d^6$) δ ppm

2.82 - 4.4 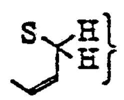 and $-O-CH_2-CH(OH)-CH_2-OH$

3.80 3H, s, $N-OCH_3$

4.76 – 5.56 2H, q, 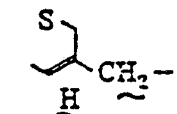

5.04 1H, d, 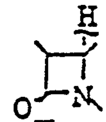

5.63 1H, dd, 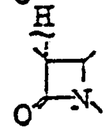

6.26 2H, s, broad

6.72 1H, s, 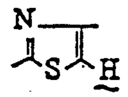

7.20 , s, broad

7.42 1H, d, 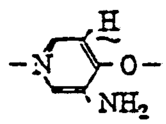

8.44 1H, s, 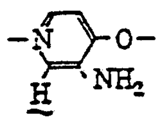

8.48 1H, d, 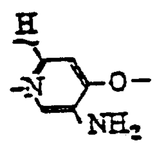

9.52 1H, d, –CONH–

IR(KBr) $cm^{-1}$

1760, 1600, 1520,

FABMS $(M+H)^+$ 580

Example 7

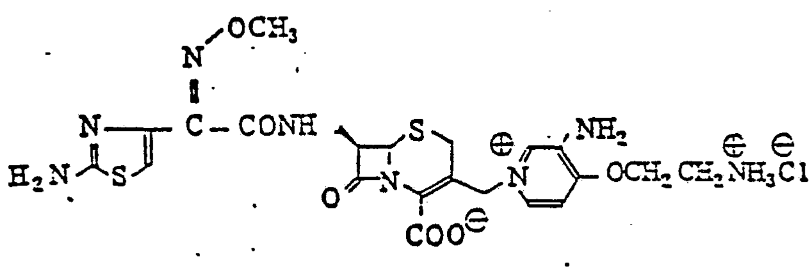

i) In 20 ml of dichloromethane was suspended 650 mg of 7-amino-3-iodomethyl-$\Delta^3$-cephem-4-carboxylic acid and after adding thereto 1.1 ml of N,O-bis(trimethylsilyl)trifluoroacetamide, the mixture was stirred for 10 minutes at room temperature to provide a solution. To the solution were added 1 ml of bis(trimethylsilyl)-trifluoroacetamide and 496 mg of 3-amino-4-[(2-t-butoxycarbonylaminoethyl)oxy]pyridine, and the mixture was stirred for 5 hours at room temperature to provide a solution containing trimethylsilyl 7-trimethylsilylamino-3-[3-amino-4-[(2-t-butoxycarbonylaminoethyl)oxy]-pyridiniomethyl]-$\Delta^3$-cephem-4-carboxylate iodide.

ii) In 10 ml of dichloromethane was suspended 847 mg of (Z)-α-(2-tritylamino-4-thiazolyl)-α-methoxyimino-acetic acid and after cooling the suspension to 3 to 4°C and then adding thereto 398 mg of phosphorus pentachloride, the mixture was stirred for 15 minutes at 3-4°C to provide a "Solution A".

A solution containing trimethylsilyl 7-trimethylsilylamino-3-[3-amino-4-[(2-t-butoxycarbonylaminoethyl)-oxy]pyridiniomethyl]-$\Delta^3$-cephem-4-carboxylate iodide obtained at 7i) above was cooled to -40°C and after adding thereto 0.77 ml of pyridine and then adding Solution A dropwise to the mixture, the temperature of the mixture was increased to -15°C over a period of 15 minutes. To the mixture were added 2 ml of water, 5 ml of tetrahydrofuran, and 4 ml of 1 N hydrochloric acid, and the mixture was stirred for 10 minutes under ice-cooling. Dichloromethane and tetrahydrofuran were distilled away under reduced

pressure. To the residue was added 100 ml of water to form precipitates, which were collected by filtration, washed with water, and dried to provide 1.74 g of a crude product of the desired compound having protective groups. To the product was added 20 ml of trifluoroacetic acid under ice-cooling and after stirring the mixture for 40 mintues at room temperature, insoluble materials were removed by filtration. To the filtrate was added 10 ml of water under ice-cooling, and the mixture was stirred for 45 minutes at room temperature. The mixture was concentrated under reduced pressure and after adding ether to the residue, the formed powder was collected by filtration to provide 1.13 g of a crude product. After adding to the product 50 ml of water and 10 ml of 1 N hydrochloric acid to dissolve it, the resultant solution was subjected to column chromatography on Diaion HP-20, and the product was eluted first with water and then with mixed solutions of water and methanol while successively changing the mixing ratio from 95:5 to 80:20. The fractions containing the desired product were collected, concentrated, and lyophilized to provide 103.4 mg of (Z)-7-[α-(2-amino-4-thiazolyl)-α-methoxyiminoacetamido]-3-[3-amino-4-(2-aminoethoxy)pyridiniomethyl]-$\Delta^3$-cephem-4-carboxylate.

o NMR ( $d^6$ − DMSO ) δ ppm

| | | |
|---|---|---|
| 2.82 − 3.7 | 6 H, | 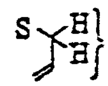 and $-OCH_2CH_2\overset{\oplus}{N}H_3\overset{\ominus}{Cl}$ |
| 3.80 | 3 H, s , | 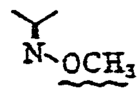 |
| 4.62 − 5.32 | 2 H, q , | 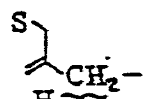 |
| 5.05 | 1 H, d , | 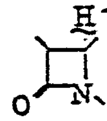 |
| 5.68 | 1 H, dd , | 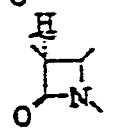 |
| 6.72 | 1 H, s , | 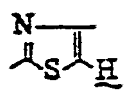 |
| 6.82 | 1 H, d, | 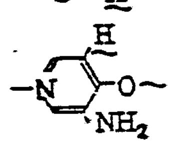 |
| 7.20 | 2 H, s , | broad |
| 7.98 | 1 H, s , | 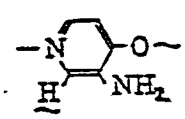 |
| 8.18 | 1 H, d , | 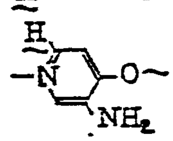 |
| 9.25 | 1 H, d, | -CONH- |

o IR (KBr) $cm^{-1}$

1760, 1620,

o FABMS $(M+H)^+$ 549

Example 8

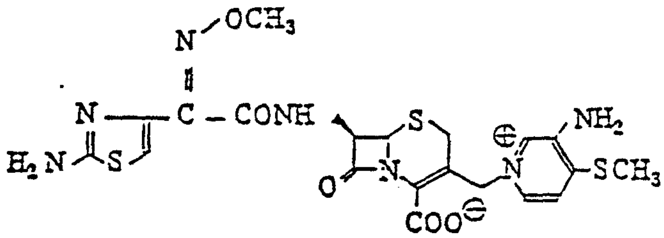

i) In 20 ml of dichloromethane was suspended 680 mg of 7-amino-3-iodomethyl-$\Delta^3$-cephem-4-carboxylic acid and after adding thereto 1.12 ml of N,O-bis(trimethylsilyl)-trifluoroacetamide, the mixture was stirred for 10 minutes at room temperature to provide a solution. To the solution was added 294 mg of 3-amino-4-methylthiopyridine, and the mixture was stirred for 4 hours at room temperature to provide a solution containing trimethylsilyl 7-trimethylsilylamino-3-(3-amino-4-methylthiopyridiniomethyl)-$\Delta^3$-cepehm-4-carboxylate iodide.

ii) In 10 ml of dichloromethane was suspended 886 mg of (Z)-$\alpha$-(2-tritylamino-4-thiazolyl)-$\alpha$-methoxyimino-acetic acid and after cooling the suspension to 3 to 4°C and adding thereto 416 mg of phosphorus pentachloride, the mixture was stirred for 15 minutes at 3-4°C to provide "Solution A".

A solution containing trimethylsilyl 7-trimethylsilylamino-3-(3-amino-4-methylthiopyridinio-

methyl)-$\Delta^3$-cephem-4-carboxylate iodide obtained at 8i) above was cooled to -40°C and after adding thereto 0.8 ml of pyridine and then addition Solution A dropwise to the mixture,the temperature of the mixture was increased to -15°C over a period of 15 minutes. To the mixture were added 2 ml of water, 5 mg of tetrahydrofuran, and 4 ml of 1 N hydrochloric acid, and the resultant mixture was stirred for 10 minutes under ice-cooling. Dichloromethane and tetrahydrofuran were distilled away under reduced pressure. To the residue was added 100 ml of water, and precipitates thus formed were collected by filtration, washed with water, and dried to provide 1.62 g of a crude product of the desired compound having protective groups. After adding to the product 20 ml of trifluoroacetic acid under ice-cooling and stirring the mixture for 15 minutes at room temperature, insoluble materials were removed by filtration. To the filtrate was added 10 ml of water under ice-cooling, and the resultant mixture was stirred for 50 minutes at room temperature. The mixture was concentrated under reduced pressure and after adding ether to the residue, the formed powder was collected by filtration to provide 0.93 g of a crude product. The product was dissolved in

0160546

50 ml of water and 5 ml of 1 N hydrochloric acid, and the solution was subjected to column chromatography on Diaion HP-20. The product was eluted first with water and then with mixed solutions of water and methanol while successively changing the mixing ratio from 95:5 to 70:30. The fractions containing the desired product were collected, concentrated, and lyophilized to provide 253 mg of (Z)-7-[α-(2-amino-4-thiazolyl)-α-methoxyimino-acetamido]-3-(3-amino-4-methylthiopyridiniomethyl)-$\Delta^3$-cephem-4-carboxylate.

NMR ($d^6$-DMSO) δppm

| | | |
|---|---|---|
| 2.88 - 3.54 | 2H, q, | 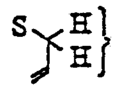 |
| 3.80 | 3H, s, | 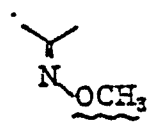 |
| 2.65 | 3H, s, | $-S\underline{CH_3}$ |
| 4.84 - 5.60 | 2H, q, | 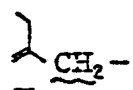 |
| 5.04 | 1H, d, | 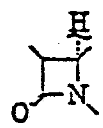 |

| | |
|---|---|
| 5.64 | 1 H, dd, 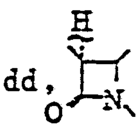 |
| 6.36 | 2 H, s, broad |
| 6.70 | 1 H, s, 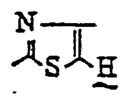 |
| 7.18 | 2 H, s, broad |
| 7.56 | 1 H, d, 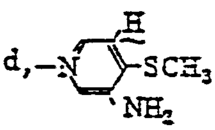 |
| 8.32 | 1 H, s, 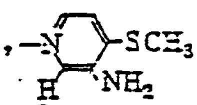 |
| 8.44 | 1 H, d, 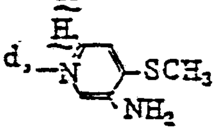 |
| 9.47 | 1 H, d, $-CONH-$ |

IR(KBr) $cm^{-1}$

1765, 1600, 1525,

FABMS $(M+H)^+$ 536

Example 9

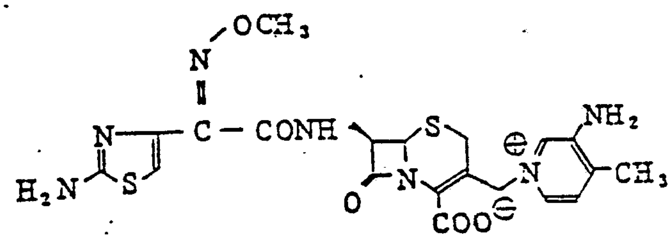

i) In 20 ml of dichloromethane was suspended 680 mg of 7-amino-3-iodomethyl-$\Delta^3$-cephem-4-carboxylic acid and after adding thereto 1.24 ml of N,O-bis(trimethylsilyl)-trifluoroacetamide, the resultant mixture was stirred for 30 minutes at room temperature to provide a complete solution. To the solution was added 216 mg of 3-amino-4-methypyridine (cf. Chemical Abstracts 63, 14805e (1965)), and the mixture was stirred for 3 hours at room temperature to provide a solution containing trimethylsilyl 7-trimethylsilylamino-3-(3-amino-4-methylpyridiniomethyl)-$\Delta^3$-cephem-4-carboxylate iodide.

ii) In 20 ml of dichloromethane was suspended 886 mg of (Z)-$\alpha$-(2-tritylamino-4-thiazolyl)-$\alpha$-methoxyimino-acetic acid and after cooling the mixture to -5°C and then adding thereto 416 mg of phosphorus pentachloride, the mixture was stirred for 15 minutes at -5 to 0°C to provide a "Solution A".

A solution containing trimethylsilyl 7-trimethylsilylamino-3-(3-amino-4-methylpyridiniomethyl)-$\Delta^3$-cephem-4-carboxylate iodide obtained at 9i) above was cooled to -40°C

and after adding thereto 0.8 ml of pyridine and then adding to the mixture Solution A, the temperature of the mixture was increased to -15°C over a period of 30 minutes. To the mixture were added 5.4 ml of 1 N hydrochloric acid, 6.7 ml of water and 6.7 ml of tetrahydrofuran, and the mixture was stirred for 10 minutes at 0°C. The reaction mixture was concentrated under reduced pressure and after adding to the residue 66 ml of water, the formed precipitates were collected by filtration.

iii) 20 ml of trifluoroacetic acid was cooled to 5°C, and the precipitates obtained at 9ii) above were added thereto while stirring. After adding 1 ml of water to the mixture, the mixture was stirred for 1 hour at room temperature. The reaction mixture was concentrated under reduced pressure and after adding to the residue 2 ml of ethanol and then adding thereto 30 ml of ether, the formed powder was collected by filtration to provide 1.03 g of a crude product. After dissolving the product in 50 ml of water and 5 ml of 1 N hydrochloric acid small amounts of insoluble materials were removed by filtration. The filtrate was subjected to column chromatography on Diaion HP-20, and the product was eluted first with water and then with mixed solutions of water and methanol while successively changing the mixing ratio up to 75:25. The fractions containing the desired product were collected by filtration, concentrated, and lyophilized to provide 200 mg of (Z)-7-[α-(2-amino-4-thiazolyl)-α-methoxyiminoacetamido]-3-(3-amino-4-methylpyridiniomethyl)-$\Delta^3$-cephem-4-carboxylic acid.

° NMR ( DMSO-$d_6$ ) δ ppm :

| | | | |
|---|---|---|---|
| 2.24 | 3 H, | s, | 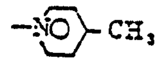 |
| 2.83 ~ 3.60 | 2 H, | q, | 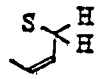 |
| 3.78 | 3 H, | s, | 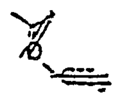 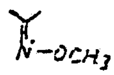 |
| 4.82 ~ 5.60 | 2 H, | q, | 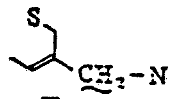 |
| 5.00 | 1 H, | d, | 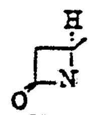 |
| 5.69 | 1 H, | dd, | 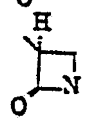 |
| 6.42 | 2 H, | broad s, | |
| 6.65 | 1 H, | s, | 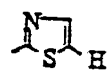 |
| 7.14 | 2 H, | broad s, | |
| 7.55 | 1 H, | d, | 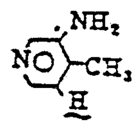 |
| 8.31 | 1 H, | d, | 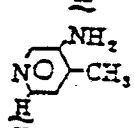 |
| 8.42 | 1 H, | s, | 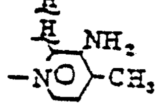 |
| 9.43 | 1 H, | d, | $-CONH-$ |

° IR ( KBr ) $cm^{-1}$ :

3300, 3175, 1765, 1650 ~ 1600, 1520

° FABMS : $(M+H)^+$ 504

Example 10

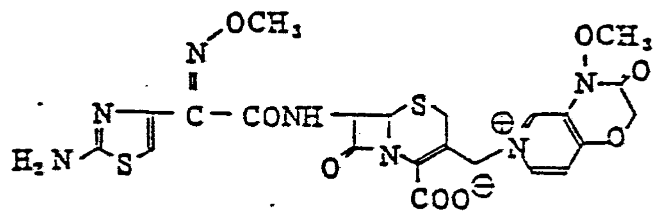

i) In 20 ml of dichloromethane was suspended 680 mg of 7-amino-3-iodomethyl-$\Delta^3$-cephem-4-carboxylic acid and after adding thereto 1.12 ml of N,O-bis(trimethylsilyl)-trifluoroacetamide, the resultant mixture was stirred for 10 minutes at room temperature to provide a solution. To the solution was added 360 mg of 4-methoxy-3-oxo-3,4-dihydro-2H-pyrido[4,3-b]-1,4-oxazine, and the mixture was stirred for 5 hours at room temperature to provide a solution containing trimethylsilyl 7-trimethylsilyl-amino-3-[[6-(4-methoxy-3-oxo-3,4-dihydro-2H-pyrido[4,3-b]-1,4-oxazinio)]methyl -$\Delta^3$-cephem-4-carboxylate iodide.

ii) In 10 ml of dichloromethane was suspended 886 mg of (Z)-$\alpha$-(2-tritylamino-4-thaizolyl)-$\alpha$-methoxyimioacetic acid and after cooling the mixture to 3 to 4°C and adding thereto 416 mg of phosphorus pentachloride, the mixture was stirred for 15 minutes at 3-4°C to provide a "Solution A".

A solution containing trimethylsilyl 7-trimethylsilylamino-3-[[6-(4-methoxy-3-oxo-3,4-dihydro-2H-pyrido[4,3-b]-1,4-oxazinio)]methyl]-$\Delta^3$-cephem-

4-carboxylate iodide obtained at 10i) above was cooled to -40°C and after adding thereto 0.8 ml of pyridine, Solution A was added dropwise to the mixture. The temperature of the mixture was increased to -15°C over a period of 15 minutes. To the reaction mixture were added 5 ml of tetrahydrofuran and 4 ml of 1 N hydrochloric acid, and the mixture was stirred for 10 minutes under ice-cooling. Dichloromethane and tetrahydrofuran were distilled away, and 100 ml of water was added to the residue. The formed precipitates were collected by filtration to provide a crude product of the compound having protective groups. To the product was added 20 ml of trifluoroacetic acid and after stirring the mixture for 15 minutes at room temperature, insoluble materials were removed by filtration. To the filtrate was added 6 ml of water, and the mixture was stirred for 1 hour at room temperature.

The reaction mixture was concentrated under reduced pressure. To the residue was added ether, and the formed powder was collected by filtration to provide 1.33 g of a crude product. To the product were added 50 ml of water and 5 ml of 1 N hydrochloric acid, and the resulting solution was subjected to column chromatography on Diaion HP-20. The product was eluted first with water and then with mixed solutions of water and methanol while changing the mixing ratio from 95:5 to 80:20. The fractions containing the desired compound were collected, concentrated, and lyophilized to provide 155 mg of (Z)-7-[α-(2-amino-4-thiazolyl)-α-methoxyimino-acetamido]-3-[[6-(4-methoxy-3-oxo-3,4-dihydro-2H-pyrido-[4,3-b]-1,4-oxazinio)]methyl $\Delta^3$-cephem-4-carboxylate.

FABMS ; $(M+H)^{+}$ 576

IR ( KBr disk ; $cm^{-1}$ ) : 1035, 1335, 1380, 1525, 1610, 1665, 1715, 1770, 3350

NMR ( DMSO - $d^{6}$ ; δ ppm )

| | | | |
|---|---|---|---|
| 2.90 ~ 3.62 , | 2H , | q , | 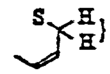 |
| 3.78 , | 3H , | s , | 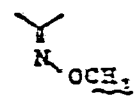 |
| 3.94 , | 3H , | s , | 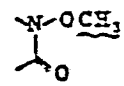 |
| 4.9 ~ 5.6 , | 2H , | q , | 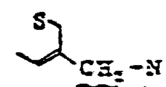 |
| 5.04 , | 1H , | d , | 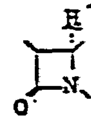 |
| 5.16 , | 2H , | s , | 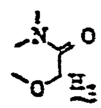 |
| 5.62 , | 1H , | dd , | 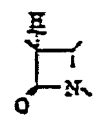 |
| 6.66 , | 1H , | s , | 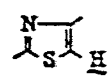 |
| 7.16 , | broad , | s | |
| 7.60 , | 1H , | d , | 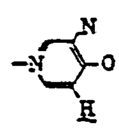 |
| 9.0 , | 1H , | d , | 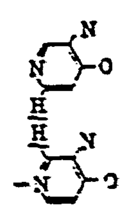 |
| 9.44 , | 1H , | s , | |

Example 11

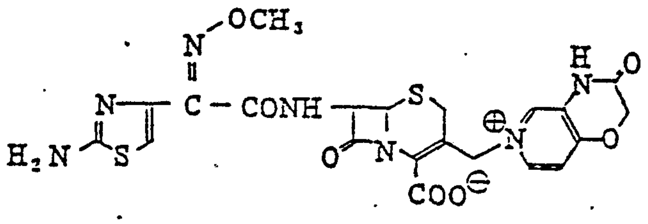

i) In 20 ml of dichloromethane was suspended 680 mg of 7-amino-3-iodomethyl-$\Delta^3$-cephem-4-carboxylic acid and after adding thereto 1.68 ml of N,O-bis(trimethylsilyl)-trifluoroacetamide, the resultant mixture was stirred for 10 minutes at room temperature to provide a solution. To the solution was added 300 mg of 3-oxo-3,4-dihydro-2H-pyrido[4,3-b]-1,4-oxazine and the mixture was stirred for 4 hours at room temperature to provide a solution containing trimethylsilyl 7-trimethylsilylamino-3-{[6-(3-oxo-3,4-dihydro-2H-pyrido[4,3-b]-1,4-oxazinio)]-methyl}-$\Delta^3$-cephem-4-carboxylate iodide. After cooling the solution to -45°C, 0.8 ml of pyridine was added thereto. To the resultant mixture was added dropwise Solution A obtained at Example 10 ii), and the temperature of the mixture was increased to -15°C over a period of 15 minutes. After adding to the reaction solution 5 ml of tetrahydrofuran and 5 ml of 1 N hydrochloric acid, the mixture was stirred for 10 minutes under ice-cooling. Dichloromethane and tetrahydrofuran were distilled away under reduced pressure. To the residue was added 100 ml of water, and the formed precipitates were collected by filtration. to provide a crude product of the compound having

protective groups. To the crude product was added 20 ml of trifluoroacetic acid under ice-cooling, and the mixture was stirred. for 15 minutes at room temperature. Insoluble materials were removed by filtration and after adding 6 ml of water to the filtrate under ice-cooling, the mixture was stirred for 1 hour at room temperature. The reaction solution was concentrated under reduced pressure and after adding enter to the residue, the formed powder was collected by filtration to provide 1.08 g of a crude product.

To the crude product were added 50 ml of water, 5 ml of 1 N hydrochloric acid, and the resulting solution was subjected to column chromatography on Diaion HP-20, and the product was eluted first with water and then mixed solutions of water and methanol while successively changing the mixing ratio from 95:5 to 80.20 The fractions containing the desired compound were collected, concentrated, and lyophilized to provide 235 mg of (Z)-7-[α-(2-amino-4-thiazolyl)-α-methoxyimino-acetamido]-3-[[6-(3-oxo-3,4-dihydro-2H-pyrido[4,3-b]-1,4-oxazinio)] methyl -$\Delta^3$-cephem-4-carboxylate.

FABMS ; $(M+H)^+$ 546

IR (KBr disk ; $cm^{-1}$): 1030, 1350, 1530, 1605, 1700, 1770, 3350

NMR ( DMSO-$d^6$ ; δppm )

| | | | |
|---|---|---|---|
| 2.92 ~ 3.58 , | 2 H , | q , | 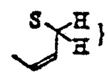 |
| 3.78 , | 3 H , | s , | 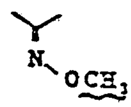 |
| 4.88 ~ 5.56 , | 2 H , | q , | 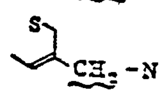 |
| 4.94 , | 2 H , | s , | 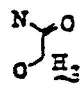 |
| 5.02 , | 1 H , | d , | 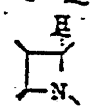 |
| 5.62 , | 1 H , | dd , | 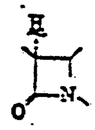 |
| 6.66 , | 1 H , | s , | 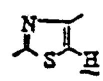 |
| 7.14 , | broad , | s | |
| 7.54 , | 1 H , | d , | 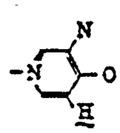 |
| 8.80 , | 1 H , | d , | 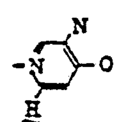 |
| 8.84 , | 1 H , | s , | 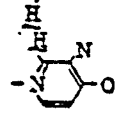 |

0160546

Example 12

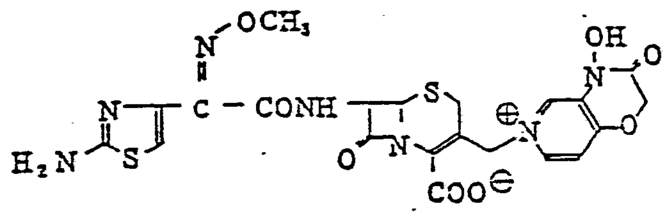

In 20 ml of dichloromethane was suspended 680 mg of 7-amino-3-iodomethyl-$\Delta^3$-cephem-4-carboxylic acid and after adding thereto 1.68 ml of N,O-bis(trimethylsilyl)-trifluoroacetamide, the mixture was stirred for 10 minutes at room temperature to provide a solution. To the solution was added 332 mg of 4-hydroxy-3-oxo-3,4-dihydro-2H-pyrido[4,3-b]-1,4-oxazine, and the mixture was stirred for 4 hours at room temperature to provide a solution containing trimethylsilyl 7-trimethylsilylamino-3-[[6-(4-hydroxy-3-oxo-3,4-dihydro-2H-pyrido[4,3-b]-1,4-oxazinio)]methyl]-$\Delta^3$-cephem-4-carboxylate iodide. This solution was cooled to -50°C and after adding thereto 0.8 ml of pyridine and further adding dropwise Solution A obtained in Example 10 ii), the temperature of the mixture was increased to -15°C over a period of 15 minutes. To the reaction mixture were added 5 ml of tetrahydrofuran and 5 ml of 1 N hydrochloric acid, and the mixture was stirred for 10 minutes under ice-cooling. Dichloromethane and tetrahydrofuran were distilled away under reduced pressure. To the residue was added 100 ml of water, and the preciptitates thus formed were collected by filtration to provide a crude product of the desired compound having protective groups. After adding to the

crude product 20 ml of trifluoroacetic acid under ice-cooling and stirring the mixture for 15 minutes at room temperature, insoluble materials were removed by filtration. To the filtrate was added 6 ml of water under ice-cooling, and the mixture was stirred for 1 hour at room temperature. The reaction mixture was concentrated under reduced pressure; to the residue was added ether and the resulting powder was collected by filtration to provide 1.1 g of crude product. The crude product was dissolved by the addition of 50 ml of water and 5 ml of 1 N hydrochloric acid, and the solution was subjected to column chromatography on Diaion HP-20. The product was eluted first with water and then with mixed solutions of water and methanol while successively changing the mixing ratio from 95:5 to 75:25. The fractions containing the desired compound were collected, concentrated, and lyophilized to provide 35.5 mg of (Z)-7-[α-(2-amino-4-thiazolyl)-α-methoxyiminoacetamido]-3-[[6-(4-hydroxy-3-oxo-3,4-dihydro-2H-pyrido[4,3-b]-1,4-oxazinio)]methyl]-$\Delta^3$-cephem-4-carboxylate.

FABMS; $(M+H)^+$ 562

NMR (DMSO-$d_6$; δ ppm)

| | | | |
|---|---|---|---|
| 2.88～3.60, | 2H, | q, | 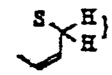 |
| 3.78, | 3H, | s, | 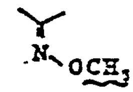 |
| 4.88～5.56, | 2H, | q, | 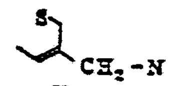 |
| 5.06, | 1H, | d, | 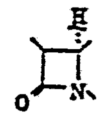 |
| 5.20, | 2H, | s, | 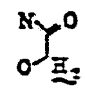 |
| 5.66, | 1H, | dd, | 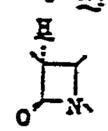 |
| 6.72, | 1H, | s, | 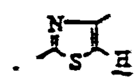 |
| 7.20, | broad | s | |
| 7.60, | 1H, | d, | 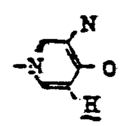 |
| 8.86, | 1H, | d, | 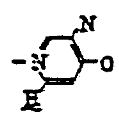 |
| 9.34, | 1H, | s, | 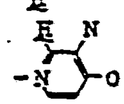 |

Example 13

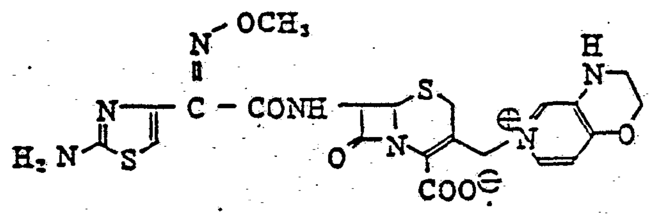

In 20 ml of dichloromethane was suspended 680 mg of 7-amino-3-iodomethyl-$\Delta^3$-cephem-4-carboxylic acid and after adding thereto 1.68 ml of N,O-bis(trimethylsilyl)-trifluoroacetamide, the mixture was stirred for 10 minutes at room temperature to provide a solution. To the solution was added 272 mg of 3,4-dihydro-2H-pyrido-[4,3-b]-1,4-oxazine, and the mixture was stirred for 3.5 hours at room temperature to provide a solution containing trimethylsilyl 7-trimethylsilylamino-3-[[6-(3,4-dihydro-2H-pyrido[4,3-b]-1,4-oxazinio)]methyl-$\Delta^3$-cephem-4-carboxylate iodide. The solution was cooled to -45°C and after adding thereto 0.8 ml of pyridine and further adding dropwise Solution A obtained in Example 10 ii), the temperature of the mixture was increased to -15°C over a period of 15 minutes. To the reaction mixture were added 5 ml of tetrahydrofuran and 5 ml of 1 N hydrochloric acid, and the mixture was stirred for 10 minutes under ice-cooling. Dichloromethane and tetrahydrofuran were distilled away under reduced pressure. To the residue was added 100 ml of

water, and the formed precipitates were collected by filtration to provide a crude product of the desired compound having protective groups. To the crude product was added 20 ml of trifluoroacetic acid under ice-cooling, and the mixture was stirred for 15 minutes at room temperature. Insoluble materials were removed by filration. To the filtrate was added 6 ml of water under ice-cooling, and the mixture was stirred for 1 hour at room temperature. The reaction mixture was concentrated under reduced pressure and after adding ether to the residue to powder it, the formed powder was collected by filtration to provide 1.15 g of a crude product. The crude product was dissolved by the addition of 50 ml of water and 5 ml of 1 N hydrochloric acid, and the solution was subjected to column chromatography on Diaion HP-20, and the product was eluted first with water and then with mixed solutions of water and methanol while successively changing the mixing ratio from 95:5 to 75:25. The fractions containing the desired compound were collected, concentrated, and lyophilized to provide 267 mg of (Z)-7-[α-(2-amino-4-thiazolyl)-α-methoxyiminoacetamido]-3-[[6-(3,4-dihydro-2H-pyrido[4,3-b]-1,4-oxazinio)]methyl]-$\Delta^3$-cephem-4-carboxylate.

FABMS $(M+H)^+$ 532

IR (KBr disk; $cm^{-1}$): 1025, 1340, 1525, 1610, 1760, 3300

NMR (DMSO-$d_6$; δ ppm)

| | | | |
|---|---|---|---|
| 2.84～3.56, | 2H, | q, | 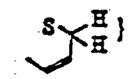 |
| 3.40, | 2H, | broad, | 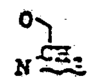 |
| 3.80, | 3H, | s, | 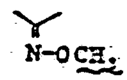 |
| 4.40, | 2H, | broad, | 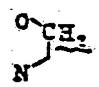 |
| 4.76～5.54, | 2H, | q, | 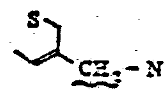 |
| 5.06, | 1H, | d, | 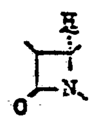 |
| 5.62, | 1H, | dd, | 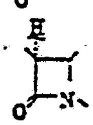 |
| 6.72, | 1H, | s, | 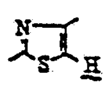 |
| 7.28, | 1H, | d, | 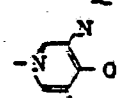 |
| 8.38, | 1H, | d, | 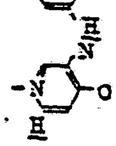 |
| 8.68, | 1H, | s, | 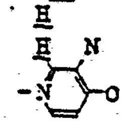 |

Example of a pharmaceutical composition:

5 g of the compound of Example 5, namely, (Z)-7-[α-(2-amino-4-thiazolyl)-α-methoxyiminoacetamido]-3-(3-amino-4-methoxypyridiniomethyl)-$\Delta^3$-cephem-4-carboxylate, was dissolved in water for injection q.s. to provide 25 ml of an aqueous solution. The solution was sterilized by filtration using a filter having pore size of 0.45 μm. The sterile solution was filled into 5 ml vials, and lyophilized. The vials were sealed to obtain intramuscular injections.

Claims:

1. A cephalosporin compound of the following general formula (I), or a pharmacologically acceptable salt thereof

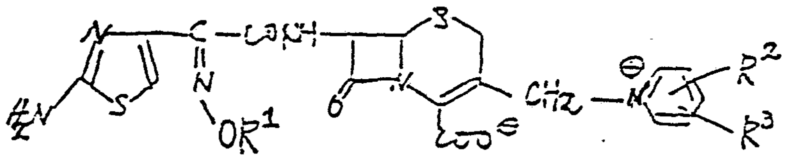

(I)

wherein $R^1$ represents a $C_1$ to $C_5$ alkyl group which may be substituted by a carboxyl group; and $R^2$ and $R^3$ are (a) the same or different and selected from an amino group, $C_1$ to $C_5$ alkyl groups, $C_1$ to $C_5$ alkylthio groups, and $C_1$ to $C_5$ alkoxy groups which may be substituted by hydroxyl group(s) and/or amino group(s), or (b) together form $-N(R^4)-A-(CH_2)_m-O-$ wherein $R^4$ represents a hydrogen atom, a hydroxyl group, or a $C_1$ to $C_5$ alkoxy group, A represents a direct linkage or a carbonyl group, and m is 1 or 2.

2. A compound according to claim 1 wherein $R^1$ is a $C_1$ to $C_5$ alkyl group; and $R^2$ and $R^3$ are the same or different and selected from amino and $C_1$ to $C_5$ alkoxy groups.

3. A compound according to claim 1 which is (Z)-7-[α-(2-amino-4-thiazolyl)-α-methoxyiminoacetamido]-3-(3-amino-4-methoxypyridiniomethyl)-$\Delta^3$-cephem-4-carboxylate or a pharmacologically acceptable salt thereof.

4. A method of preparing a compound according to claim 1 which comprises reacting

7-amino-3-halogenomethyl-$\Delta^3$-cephem-4-carboxylic acid II

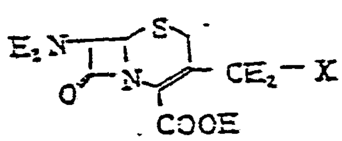

II

(wherein X represents a halogen atom)

with N,O-bis(tri-loweralkylsilyl)trifluoroacetamide III

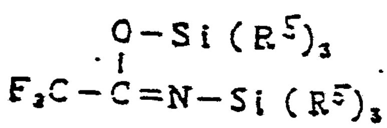

(III)

wherein $R^5$ represents a $C_1$ to $C_5$ alkyl group

and substituted pyridine compound IV

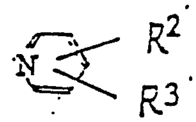

(IV)

and then reacting the formed compound with substituted oxyiminothiazolyl acetic acid compound V or reactive derivative thereof,

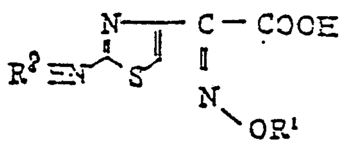

V

(wherein $R^8$ represents a hydrogen atom or a protective group for an amino group) and then, if necessary, releasing any protective group.

5. A method of preparing a compound according to claim 1 which comprises reacting

7-amino-3-cephem derivative VI with substituted oxyiminothiazolylacetic acid V or reactive derivative thereof and then, if necessary, releasing any protective group(s);

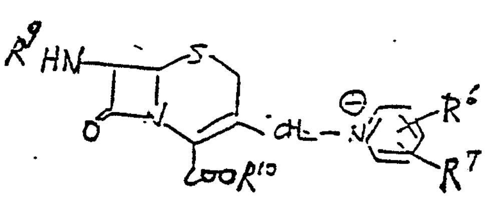

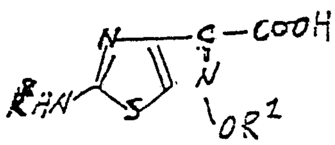

(VI) (V)

6 . A method of preparing a compound according to claim 1 which comprises either (i) reacting compound VII which may have protective group(s) for the amino group and/or the carboxy group, directly with substituted pyridine compound IV ;

or (ii) converting compound VII to the 3-halogenomethylcephalosporin derivative of formula VIII and then reacting this with substituted pyridine compound IV

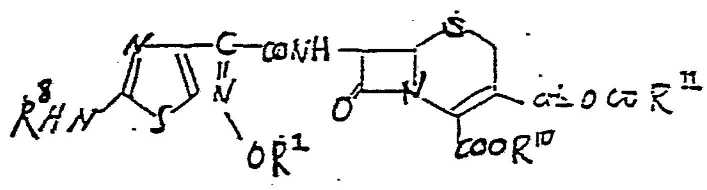

(VII)

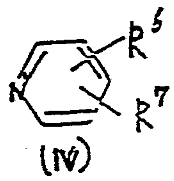

(IV)

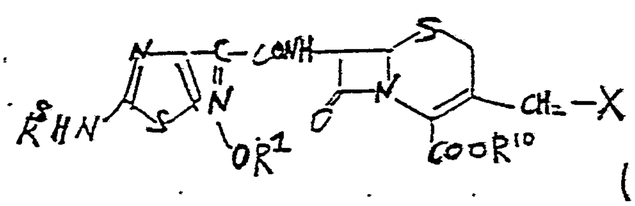

(VIII)

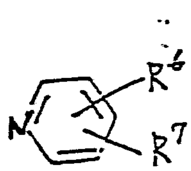

(IV)

7. A pharmaceutical composition containing a compound according to claim 1 as an active ingredient

8. A pharmaceutical composition according to claim 7 which is an injection.